# EUROPEAN PATENT APPLICATION

(11) **EP 3 677 177 A1**
(43) Date of publication of application: **08.07.2020**
(21) Application number: 18867563.1
(22) Date of filing: 15.06.2018
(51) Int. Cl.: A61B 5/0408, A61B 5/0404, A61B 5/0476, A61B 5/0478, A61B 5/0488

(54) **GARMENT, CONDUCTIVE COMPOSITE THREAD, AND DISPOSABLE GARMENT**

(30) Priority: 20.10.2017 JP 2017203641
(71) Applicant: Mitsufuji Corporation, Kyoto 619-0237 (JP)
(72) Inventor: MITERA, Hideyuki, Kyoto 619-0237 (JP); AMADUTSUMI, Koji, Kyoto 619-0237 (JP); SUMIYOSHI, Yuji, Kyoto 619-0237 (JP)
(74) Representative: Haseltine Lake Kempner LLP
(86) International application number: PCT/JP2018/022888
(87) International publication number: WO 2019/077799

(57) **Abstract**

A garment configured to detect a biopotential to ease discomfort to the wearer thereof is provided. Insulation area (10) is a piece of cloth with thickness t10, and has contact surface (100) that comes into contact with the skin of wearer (P). Electrode area (11) is a piece of cloth with thickness t11, and has contact surface (110) that comes into contact with the skin of wearer (P). Electrode area (11) is formed by weaving a thread that contains a piece of conductive fiber, formed by adding metal such as silver or copper, or a conductive material such as conductive polymer, to a piece of non-conductive fiber, such that a step (difference in height) is not formed between electrode area (11) and insulation area (10).

## Description

### Technical Field

The present invention relates to a garment that can detect a biopotential, a conductive composite thread, and a disposable garment.

### Background Art

A system that detects the biopotential of the wearer of a garment, using an electrode provided on the garment, has been developed. Patent Literature 1 discloses a bioelectrical signal monitoring garment that is provided with a close-contact means that fixes a portion around the installation area of an electrode, of a piece of stretch fabric, in a stretched state, and brings the electrode into close contact with a living body.

### Citation List

### Patent Literature

Patent Literature 1: JP 2015-100673A

### Summary of Invention

### Technical Problem

According to the technology disclosed in Patent Literature 1, a biometric electrode is installed in a predetermined area of a piece of fabric, and therefore the surface of the biometric electrode is closer to the living body than the surface of the piece of fabric is, due to the thickness thereof. That is to say, the bioelectrical signal monitoring garment disclosed in Patent Literature 1 is configured such that there may be a step (difference in height) between an area in which the biometric electrode is installed and the other area. Furthermore, according to the Patent Literature 1, the close-contact means brings the biometric electrode into close contact with the living body, and therefore the corners of the aforementioned step are likely to rub against the living body and cause discomfort.

The present invention aims to provide a garment that detects a biopotential and eases discomfort to the wearer thereof.

### Solution to Problem

A garment according to claim 1 of the present invention is a garment that includes: an insulation area formed by weaving a piece of insulating fabric that insulates electricity; and an electrode area formed by weaving a thread that contains a piece of conductive fiber made by adding a conductive material to a piece of non-conductive fiber, such that a step is not formed between the electrode area and the insulation area, wherein the electrode area is provided at a position that comes into contact with the body of a wearer so that a biopotential can be detected when a wearer wears the garment.

A garment according to claim 2 of the present invention is the garment according to claim 1, wherein the thread that contains the piece of conductive fiber is a conductive composite thread formed by winding the piece of conductive fiber around a piece of elastic fiber that has a stretch ratio of at least 50%.

A garment according to claim 3 of the present invention is the garment according to claim 2, wherein the piece of insulating fabric is an insulating composite thread formed by winding the piece of non-conductive fiber around the piece of elastic fiber.

A garment according to claim 4 of the present invention is the garment according to claim 3, wherein the winding number of the piece of non-conductive fiber in the insulating composite thread is the same as the winding number of the piece of conductive fiber in the conductive composite thread.

A garment according to claim 5 of the present invention is the garment according to any one of claims 1 to 4, wherein the electrode area is provided at two positions that are separated from each other by the insulation area.

A garment according to claim 6 of the present invention is the garment according to claim 5, further including: a receiver that receives the biopotential detected by each of the electrode areas provided at the two positions; wires that respectively extend from the electrode areas; two terminals that are respectively provided at end portions of the wires, and are connected to the receiver; and a fixing member that is provided away from a line that connects the two terminals to each other, and fixes the receiver to the insulation area.

A garment according to claim 7 of the present invention is the garment according to any one of claims 1 to 6, wherein the electrode area is provided at a position at which a skin between the chest and the abdomen of the wearer contacts with the electrode area.

A conductive composite thread according to claim 8 of the present invention is a conductive composite thread formed by winding a piece of conductive fiber, formed by adding silver to a piece of non-conductive fiber, around a piece of elastic fiber that has a stretch ratio of at least 50%.

A conductive composite thread according to claim 9 of the present invention is the conductive composite thread according to claim 8, wherein the non-conductive fiber is nylon and the elastic fiber is polyurethane.

A disposable garment according to claim 10 of the present invention is a disposable garment including: a piece of non-woven cloth that has a pocket and an opening; an electrode that is formed from the conductive composite thread according to claim 8 or 9, and is bonded to the piece of non-woven cloth; a receiver that is to be housed in the pocket; and a wire that connects the electrode to the receiver, wherein the disposable garment is to be worn by a wearer such that a side, to which the electrode is bonded, of the piece of non-woven cloth faces the front surface of the body of the wearer, and the head of the wearer passes through the opening.

A disposable garment according to claim 11 of the present invention is the disposable garment according to claim 10, wherein the pocket is located on the left side of the front surface when the disposable garment is worn by the wearer.

A disposable garment according to claim 12 of the present invention is the disposable garment according to claim 10 or 11, wherein the pocket has an inlet port on the lower edge side when the disposable garment is worn by the wearer, and the receiver is to be inserted into the pocket from the inlet port.

### Effects of Invention

The invention according to the present application provides a garment that detects a biopotential and eases discomfort to the wearer thereof.

### Brief Description of Drawings

FIG. 1 is a diagram showing an example of detection system 9 according to an embodiment of the present invention.
FIG. 2 is a diagram showing a configuration of transceiver 2.
FIG. 3 is a diagram showing an external appearance of transceiver 2.
FIG. 4 is a diagram showing an example of a configuration of terminal 3.
FIG. 5 is a front view of a detection area of garment 1.
FIG. 6 is a cross-sectional view of the detection area of garment 1.
FIG. 7 is a diagram showing a configuration of conductive composite thread 111.
FIG. 8 is a diagram showing an example of electrode area 11 formed by weaving conductive composite threads 111.
FIG. 9 is a diagram showing garment 8 in which a surface that comes into contact with wearer P has a step (difference in height).
FIG. 10 is a diagram showing an example of detection system 9a that includes disposable garment 4 according to a modification.
FIG. 11 is a diagram showing the shape of disposable garment 4.
FIG. 12 is a cross-sectional view of a detection area of disposable garment 4.
FIG. 13 is a cross-sectional view of disposable garment 4 formed so as not to have a step (difference in height).

### Description of Embodiments

### Embodiment

### Configuration of Detection System

In the following description, the "inside" of garment 1 or the like is the side close to a body of user a wear (wearer) P, and the "outside" thereof is the side farther from the body of wearer P.

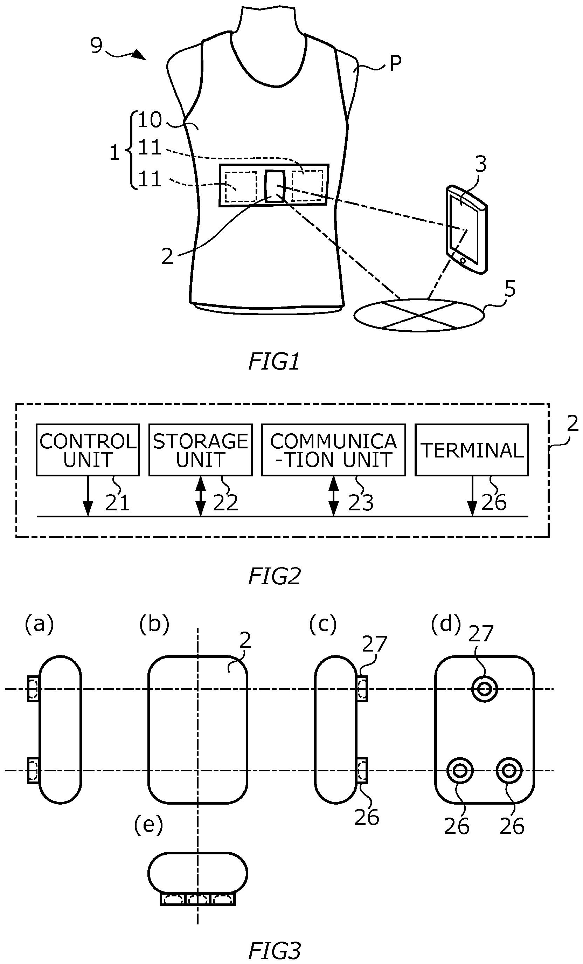

FIG. 1 is a diagram showing an example of detection system 9 according to the present embodiment. As shown in FIG. 1, detection system 9 includes garment 1, transceiver 2, and terminal 3. Detection system 9 according to the present embodiment is a system that detects a biopotential from wearer P of garment 1, and provides the user with biometric information indicating the biopotential via terminal 3.

Biometric information is information that can be obtained from the detected biopotential, examples of which include electrocardiographic information, heart rate, respiration, pulse waves, body temperature, myoelectricity, brain waves, an eyeball potential, blood pressure, sweat amount, blood sugar level, humidity, and so on, but may be any information that can be obtained upon the body of wearer P touching electrode areas. In the following description, biometric information is electrocardiographic information.

Garment 1 is a garment to be worn by wearer P, and is provided with insulation area 10 that is formed by weaving a piece of insulating fabric that insulates electricity, and electrode areas 11 for detecting the biopotential of wearer P. Electrode areas 11 are provided at positions that come into contact with the body of wearer P so that the biopotential can be detected when wearer P wears garment 1.

In the present embodiment, the biometric information to be detected is electrocardiographic information, and therefore a myoelectric potential is noise. For example, if electrode areas 11 are provided at a position near the pectoralis major, the rectus abdominis, or the like, it is difficult to measure electrocardiographic information due to the influence of a myoelectric potential. Therefore, in the present embodiment, electrode areas 11 are provided so as to be located between the chest and abdomen of wearer P and so as to be unlikely to be affected by the myoelectric potential of the pectoralis major, the rectus abdominis, or the like. That is to say, electrode areas 11 are provided at a position that comes into contact with the skin between the chest and abdomen of wearer P. In the following description, the area between the chest and abdomen of wearer P is referred to as a "detection area".

Electrode areas 11 are respectively provided on the left side and the right side of the detection area, and a position therebetween, i.e. a portion corresponding to the epigastrium of wearer P, is provided with an area to which transceiver 2 is attached.

Transceiver 2 is a device that receives biometric information of wearer P detected by electrode areas 11, and transmits a signal (a biometric signal) corresponding to the biometric information to terminal 3. Transceiver 2 uses short-range wireless communication conforming to the IEEE 802.15.1 standards, for example, to transmit a biometric signal to terminal 3.

Terminal 3 is, for example, a smartphone, a tablet PC, or the like, and is a device that provides the user with the biometric information of wearer P based on a signal received from transceiver 2.

If terminal 3 is connected to transceiver 2 via communication network 5, detection system 9 may include communication network 5. Communication network 5 is a network that mediates the exchange of information between transceiver 2 and terminal 3, such as the Internet. The biological signal transmitted from transceiver 2 may be stored in information processing apparatuses such as a cloud system connected to communication network 5, and provided to terminal 3 from these information processing apparatuses.

### Configuration of Transceiver

FIG. 2 is a diagram showing a configuration of transceiver 2. Control unit 21 includes a CPU (Central Processing Unit), a ROM (Read Only Memory), and a RAM (Random Access Memory), and controls the components of transceiver 2 as a result of the CPU reading out and executing computer programs (hereinafter simply referred to as programs) stored in the ROM and storage unit 22.

Storage unit 22 is a storage means such as a solid state drive, and stores various programs that are to be read by the CPU of control unit 21.

Communication unit 23 is a communication circuit that is wirelessly connected to terminal 3. Transceiver 2 transmits a biometric signal that indicates biometric information detected by garment 1, to terminal 3 via communication unit 23.

Terminals 26 are terminals that are connected to wires 12 drawn from electrode areas 11 of garment 1, described below. Terminals 26 are, for example, hooks that respectively have recesses. Transceiver 2 receives biometric information from garment 1 via terminals 26, and transmits a biometric signal that indicates the received biometric information, to terminal 3 via communication unit 23.

FIG. 3 is a diagram showing an external appearance of transceiver 2. FIG. 3(a) shows transceiver 2 viewed from the right side of wearer P, and FIG. 3(c) shows that viewed from the left side of wearer P. FIG. 3(b) shows transceiver 2 viewed from the front side (outside) of wearer P, FIG. 3(d) shows that viewed from the wearer P's body side (inside), and FIG. 3(e) shows transceiver 2 viewed from below wearer P.

As shown in FIG. 3, transceiver 2 is provided with fixing member 27 in addition to two terminals 26. Fixing member 27 is located away from a line that connects two terminals 26. Fixing member 27 is a structure for fixing transceiver 2 to garment 1, and is, for example, a hook with a recess.

### Configuration of Terminal

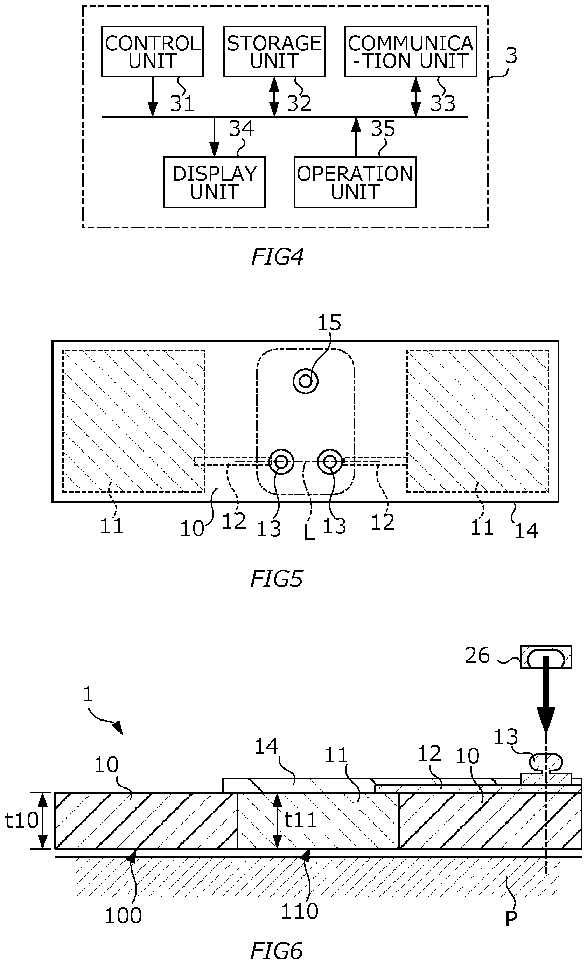

FIG. 4 is a diagram showing an example of a configuration of terminal 3. Terminal 3 includes control unit 31, storage unit 32, communication unit 33, display unit 34, and operation unit 35.

Control unit 31 includes a CPU, a ROM, and a RAM, and controls the components of terminal 3 as a result of the CPU reading out and executing programs stored in the ROM and storage unit 32.

Storage unit 32 is a storage means such as a solid state drive, and stores various programs that are to be read by the CPU of control unit 31.

Communication unit 33 is a communication circuit that is wirelessly connected to transceiver 2. Terminal 3 receives a biometric signal that indicates the biometric information detected by garment 1, from transceiver 2 via communication unit 33.

Operation unit 35 is provided with operation elements such as an operation button for inputting various instructions, and accepts an operation performed by the user and supplies control unit 31 with a signal corresponding to the content of the operation. Operation unit 35 may include a touch panel that detects the user's finger or an operation member such as a stylus pen.

Display unit 34 includes a display screen such as a liquid crystal display, and displays images under the control of control unit 31. A transparent touch panel of operation unit 35 may be laid over the display screen.

Display unit 34 displays an image corresponding to biometric information under the control of control unit 31. For example, display unit 34 displays an electrocardiogram or an electrocardiographic waveform based on biometric information indicating the electrocardiographic information acquired from transceiver 2. Also, display unit 34 may display the RRI (R-R Interval), which is fluctuating time-series data regarding heartbeat, or heart rate, using characters.

Note that terminal 3 does not necessarily display biometric information using display unit 34, in order to provide the user with biometric information. For example, terminal 3 may include a sound emitting device such as a speaker that provides the user with biometric information by outputting a sound.

### Configuration of Garment

FIG. 5 is a front view of the detection area of garment 1. Electrode areas 11, which are separated from each other by insulation area 10, are respectively provided on the left side and the right side of the detection area. Wires 12 respectively extend from electrode areas 11 provided at these two positions.

If electrode areas 11 and wires 12 are exposed to the outside, they may cause a short circuit as a result of coming into contact with a conductive object. To prevent such a short circuit, the outer sides of electrode areas 11 and wires 12 are covered by insulation member 14. Insulation member 14 is, for example, a urethane sheet or the like that can be bonded by applying heat and pressure thereto, and is bonded to electrode areas 11 of garment 1 and insulation area 10 therearound.

End portions of two wires 12 are respectively provided with terminals 13. These terminals 13 penetrate through insulation member 14 and are exposed to the outside. Terminals 13 are terminals that are connected to terminals 26 and constitute a communication path to transmit signals to transceiver 2. For example, if terminals 26 are hooks that respectively have recesses, terminals 13 are hooks that respectively have protrusions that can be fitted into the recesses.

Fixing member 15 is a component for fixing transceiver 2 to garment 1, and is fixed onto insulation member 14 by, for example, being sewn thereto. For example, if fixing member 27 of transceiver 2 is a hook that has a recess, fixing member 15 is a hook that has a protrusion that can be fitted into the recess.

As shown in FIG. 5, fixing member 15 is located away from line L that connects two terminals 13. This is because, if fixing member 15 is provided on an extension of line L, transceiver 2 will rotate about line L, and transceiver 2 is unlikely to be stably fixed to garment 1. Fixing member 15 is located away from line L, and thus transceiver 2 is fixed to insulation area 10 of garment 1.

FIG. 6 is a cross-sectional view of the detection area of garment 1. Insulation area 10 is a piece of cloth with thickness t10, and has contact surface 100 that comes into contact with the skin of wearer P. Electrode area 11 is a piece of cloth with thickness t11, and has contact surface 110 that comes into contact with the skin of wearer P.

Electrode areas 11 are formed by weaving threads that contain a conductive fiber made by adding a conductive metal material such as silver, copper, stainless steel, nickel or aluminum, or a conductive non-metal material such as carbon or a conductive polymer, to fiber with non-conductive properties (referred to as non-conductive fiber) such that a step (difference in height) is not formed between electrode areas 11 and insulation area 10. Therefore, as shown in FIG. 6, there is no step (difference in height) between contact surface 100 and contact surface 110. Therefore, the boundary between contact surface 100 and contact surface 110 will almost never rub against the skin of wearer P, and the boundary portion is unlikely to cause discomfort to wearer P.

The step (difference in height) between contact surface 100 and contact surface 110 is not necessarily perceived by wearer P. For example, a step that cannot be perceived by wearer P is a step of several millimeters may exist. For example, a seamless flat knitting machine or the like that is controlled by a computer is used as a means for weaving electrode areas 11 integrally with insulation area 10.

### Configuration of Conductive Composite Thread

As described above, insulation area 10 is formed by weaving a piece of insulating fabric that insulates electricity. Electrode areas 11 are formed by weaving threads that contain a conductive fiber, with a piece of insulating fabric that constitutes insulation area 10, such that a step (difference in height) is not formed between electrode areas 11 and insulation area 10. Here, conductive composite thread 111 will be described as an example of a thread that contains a conductive fiber.

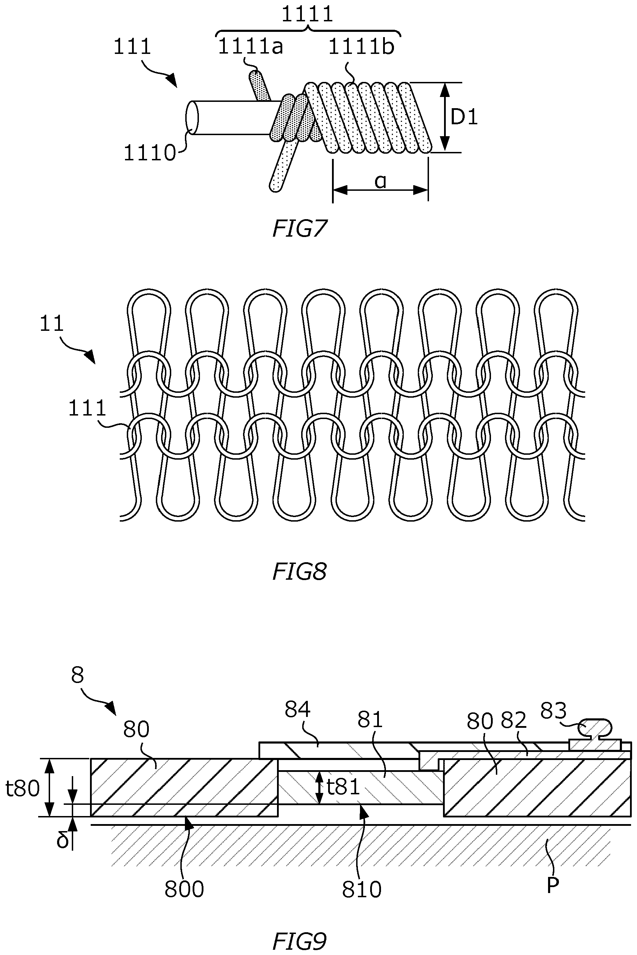

FIG. 7 is a diagram showing a configuration of conductive composite thread 111. Conductive composite thread 111 is a composite thread formed by winding conductive fiber 1111 around elastic fiber 1110. Elastic fiber 1110 is a piece of fiber that has a stretch ratio of at least 50%, and is polyurethane, for example. Conductive fiber 1111 is a piece of fiber formed by adding a conductive material to a non-conductive fiber, and is, for example, a thread obtained by plating silver on nylon. In order to stably detect a biopotential, the ratio of silver to nylon need only be no less than 10 percent by weight, but it is more preferable that the ratio is no less than 30 percent by weight.

Note that elastic fiber 1110 is not limited to polyurethane, and may be, for example, an elastic polyester, a natural rubber fiber, a thermally-shrunk nylon, or the like. Also, the non-conductive fiber is not limited to nylon, and may be a natural fiber such as cellulose or raw silk, or another synthetic fiber, as long as the conductivity thereof is below a predetermined threshold value. If a natural fiber is used as the non-conductive fiber, garment 1 can be worn by wearer P even if wearer P is allergic to synthetic fibers.

Also, the method for adding a conductive material to the non-conductive fiber is not limited to plating, and may be, for example, vapor deposition, sputtering, metal sheet bonding, impregnation, or the like. Conductive fiber 1111 may be formed by, for example, impregnating an acrylic fiber with copper sulfide.

Conductive composite thread 111 is formed by doubly winding conductive fiber 1111 around elastic fiber 1110 that serves as a core thread. That is to say, conductive composite thread 111 is formed as a double covered yarn.

Conductive composite thread 111 is formed by first winding lower winding thread 1111a, which is conductive fiber 1111, around elastic fiber 1110 at pitch α, and thereafter winding upper winding thread 1111b, which is conductive fiber 1111, on the lower winding thread 1111a, at pitch α, in the reverse direction. Thus, conductive composite thread 111 with diameter D1 and pitch α is formed. Pitch α is the number of times conductive fiber 1111 is wound per unit length (hereinafter also referred to as a winding number).

FIG. 8 is a diagram showing an example of electrode area 11 formed by weaving conductive composite thread 111. For example, electrode area 11 is formed in the shape of a piece of cloth by weaving conductive composite thread 111 in the pattern shown in FIG. 8. Conductive composite thread 111 is formed by using elastic fiber 1110 in which the core thread has a stretch ratio of at least 50%, and therefore electrode area 11 per se is easily stretchable, and can easily come into close contact with the skin of wearer P.

Insulation area 10 is formed adjacent to electrode area 11 by weaving a piece of insulating fabric. This piece of insulating fabric may be an insulating composite thread formed by winding a piece of non-conductive fiber around a piece of elastic fiber 1110.

In this case, the insulating composite thread that constitutes insulation area 10 may be formed by winding a non-conductive fiber around elastic fiber 1110 such that the diameter thereof equals diameter D1 of conductive composite thread 111 shown in FIG. 7. The insulating composite thread may be formed by winding a piece of non-conductive fiber around elastic fiber 1110 at pitch α the same number of times as the number of times conductive fiber 1111 is wound around elastic fiber 1110 in conductive composite thread 111. In other words, the winding number of the non-conductive fiber around the elastic fiber in the insulating composite thread may be the same as the winding number of the conductive fiber around the elastic fiber in the conductive composite thread.

As the winding number and the pitch of the insulating composite thread that constitutes insulation area 10 and conductive composite thread 111 that constitutes electrode area 11 are set to be the same, it is unlikely that wearer P will perceive a step at the boundary between insulation area 10 and electrode area 11. As with conductive composite thread 111, the insulating composite thread is formed using double-covered yarn instead of single-covered yarn. Note that when conductive composite thread 111 is formed using single-covered yarn, the insulating composite thread may be formed using a piece of single-covered yarn so that the insulating composite thread is formed using the same method as conductive composite thread 111.

In particular, it is preferable that the elastic fiber and the non-conductive fiber used to form the insulating composite thread and conductive composite thread 111 are made of the same material and have the same diameter. As a result, the only difference between the insulating composite thread and conductive composite thread 111 is whether or not a conductive material has been added to a non-conductive fiber, and there is almost no influence on the volumes thereof. By weaving the insulating composite thread and conductive composite thread 111 with such configurations integrally with each other, insulation area 10 and electrode areas 11 are seamlessly connected and garment 1 does not have a step.

Note that the winding numbers do not necessarily have to be exactly the same as long as wearer P cannot perceive the difference. For example, the ratio between the number of times conductive fiber 1111 is wound around elastic fiber 1110 in conductive composite thread 111 and the number of times a non-conductive fiber is wound around elastic fiber 1110 in the insulating composite thread may be within the range of ±20%, and preferably within the range of ±5%.

FIG. 9 is a diagram showing garment 8 in which a surface that comes into contact with wearer P has a step. Garment 8 includes insulation area 80, electrode area 81, wire 82, terminals 83, and insulation member 84. Electrode area 81 is formed by, for example, weaving a thread that contains a conductive fiber, with insulation area 80. However, the thread that constitutes insulation area 80 and the thread that constitutes electrode area 81 do not have the same configuration (whether or not it is a single piece of fabric or a composite thread), diameter, winding number, pitch, or the like. Therefore, a step δ that can be perceived by wearer P is formed between contact surface 810 on which electrode area 81 comes into contact with the skin of wearer P and contact surface 800 on which insulation area 80 comes into contact with the skin of wearer P.

With such a configuration, the boundary between insulation area 80 and electrode area 81 may rub against the skin of wearer P and causes discomfort to wearer P. Also, electrode area 81 is likely to become detached from the skin of wearer P, and therefore the detection of a biopotential is unstable.

As described above, garment 1 according to the present embodiment is formed such that a step (difference in height) that can be perceived by wearer P is not present between insulation area 10 and electrode areas 11. Therefore, garment 1 is unlikely to cause discomfort to wearer P, and the detection of a biopotential is unlikely to be unstable.

### Modifications

While an embodiment has been described above, this embodiment may be modified as described below. Also, the modifications described below may be combined.

### Modification 1

In the above-described embodiment, insulation area 10 in garment 1 is formed by weaving an insulating composite thread. However, insulation area 10 may be formed from non-woven cloth. Non-woven cloth is relatively inexpensive and can be mass-produced, and therefore a garment in which the insulation area is formed from non-woven cloth may be used as a disposable garment.

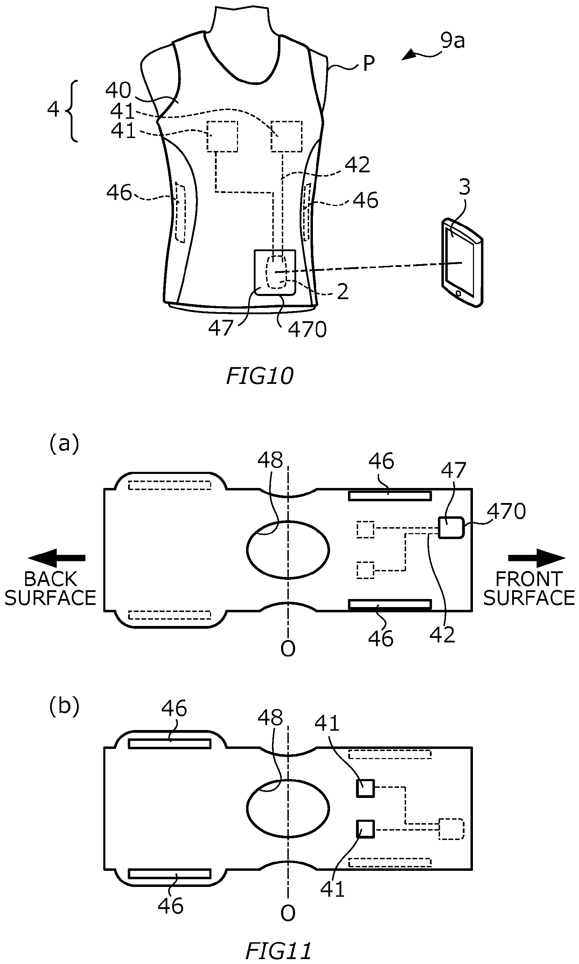

FIG. 10 is a diagram showing an example of detection system 9a that includes disposable garment 4 according to a modification. Detection system 9a includes garment 4 instead of garment 1 according to the above-described embodiment, in addition to transceiver 2 and terminal 3.

Disposable garment 4 includes insulation area 40 that is formed from a piece of non-woven cloth, and electrode area 41 that is an electrode formed from conductive composite thread 111 described above and is bonded to the piece of non-woven cloth. Hook-and-loop fasteners 46 are provided on the left and right side surfaces of wearer P. These hook-and-loop fasteners 46 are used to attach and fix different portions of a piece of non-woven cloth thereto, and form disposable garment 4. Hook-and-loop fasteners 46 are used to form disposable garment 4, and thus disposable garment 4 is less likely to place strain on the skin of wearer P, and can be easily detachable, and make disposable garment 4 durable enough to be worn a plurality of times.

Pocket 47 that has an inlet port 470 is provided on insulation area 40. Pocket 47 may be provided at any position on the outer side of disposable garment 4, but is preferably provided on the front surface of the body of wearer P. Also, it is particularly preferable that pocket 47 is located on the left side of the front surface when disposable garment 4 is worn by wearer P. When disposable garment 4 is worn by wearer P, if pocket 47 is located on the left side of the front surface of the body, less stress is placed on wearer P when they roll over in bed. In particular, when wearer P is sleeping on their right side so that the direction of their stomach matches the direction of gravity, if pocket 47 is located on the left side of the front surface, pocket 47 is less likely to move to a position under the body of wearer P.

Pocket 47 is formed by, for example, sewing three sides of a piece of non-woven cloth, which has been cut into a rectangle, to a piece of non-woven cloth that constitutes insulation area 40. The sides to which pocket 47 is sewn are the top, right, and left sides when seen from wearer P, and the one side on the bottom is not sewn and is left open so as to constitute inlet port 470. That is to say, pocket 47 has inlet port 470 on the lower edge side when disposable garment 4 is worn by wearer P. Transceiver 2 is inserted upward into pocket 47 via inlet port 470. Since inlet port 470 is provided on the lower edge side, transceiver 2 is prevented from being mistakenly removed from pocket 47 by wearer P.

Electrode areas 41 are connected to transceiver 2 by wires 42. Thus, biometric information that is based on the biopotential of wearer P detected by electrode areas 41 is received by transceiver 2 via wires 42, and is transmitted from transceiver 2 to terminal 3.

FIG. 11 is a diagram showing a piece of non-woven cloth that constitutes disposable garment 4. Disposable garment 4 is formed by adding electrode areas 41, wires 42, and terminals 43 (omitted from FIG. 11) to a piece of non-woven cloth that has pocket 47 and opening 48. FIG. 11(a) shows a surface of the piece of non-woven cloth, the surface being located on the outer side when the piece of non-woven cloth is used as disposable garment 4. FIG. 11(b) shows a surface of the piece of non-woven cloth, the surface being located on the inner side when the piece of non-woven cloth is used as disposable garment 4. As shown in FIG. 11(b), two electrode areas 41 are formed on a detection area between the chest and abdomen of wearer P, on the inner side of the piece of non-woven cloth.

In FIG. 11, the right side of center line O of opening 48 is the front side of wearer P, and the left side of center line O is the back side of wearer P. The piece of non-woven cloth shown in FIG. 11(a) is mountain-folded along center line O, the head of wearer P is passed through opening 48, hook-and-loop fasteners 46 are attached to each other, and thus disposable garment 4 is worn by wearer P. In this way, it is possible to make such a piece of non-woven cloth wearable into disposable garment 4 by folding the single piece of non-woven cloth once and attaching the hook-and-loop fasteners on each of the two side surfaces to each other. Therefore, it is unnecessary to perform a sewing process or the like, and manufacturing costs are reduced.

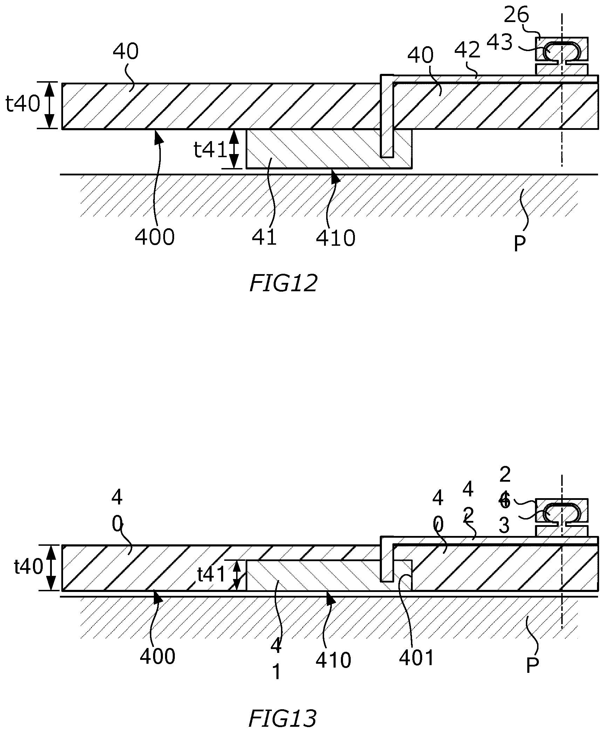

FIG. 12 is a cross-sectional view of the detection area of disposable garment 4. Terminals 43 have the same configurations as terminals 13 according to the embodiment, and are connected to terminals 26. Insulation area 40 is a piece of cloth with thickness t40, and has contact surface 400 that comes into contact with the skin of wearer P. Electrode areas 41 are pieces of cloth with thickness t41, and each have contact surface 410 that comes into contact with the skin of wearer P.

Electrode areas 41 are bonded to a piece of non-woven cloth that is insulation area 40. Therefore, contact surface 410 is closer to wearer P than contact surface 400 is, by thickness t41 of electrode area 41. In this case, this step may be perceived by wearer P. However, insulation area 40 is formed from a piece of non-woven cloth, and therefore easily deforms when compressed. Therefore, the aforementioned step may be absorbed by the deformation of the piece of non-woven cloth.

Insulation area 40 may be processed in advance. FIG. 13 is a cross-sectional view of disposable garment 4 formed such that there is no step between insulation area 40 and electrode area 41. In insulation area 40, recess 401 has been formed in advance in an area where electrode area 41 is to be provided, so as to have a depth that is substantially equal to thickness t41. This recess 401 is formed by, for example, scraping a portion of a piece of non-woven cloth, using a cutter or the like. Electrode area 41 that has thickness t41 is fitted into, and is bonded to, this recess 401. As a result, there is substantially no step between contact surface 400 and contact surface 410.

### Modification 2

In the above-described embodiment, transceiver 2 is connected to terminal 3 via wireless communication and transmits biometric information to terminal 3. However, transceiver 2 may be a receiver without a transmission function. For example, transceiver 2 may be a data logger that stores the received biometric information in storage unit 22.

### Modification 3

In the above-described embodiment, the insulating composite thread is formed by winding a piece of non-conductive fiber around elastic fiber 1110 so as to have the same diameter D1 as conductive composite thread 111. However, the insulating composite thread and conductive composite thread 111 may have different diameters as long as garment 1 is formed such that there is no step between insulation area 10 and electrode areas 11.

The insulating composite thread may be formed by winding a piece of non-conductive fiber around elastic fiber 1110 a number of times different from the number of times conductive fiber 1111 is wound around elastic fiber 1110 in conductive composite thread 111. For example, even if an insulating composite thread with the winding number different from that of conductive composite thread 111 is used, it is possible not to form a gap between insulation area 10 and electrode areas 11 by employing a weaving pattern or the like different from that of electrode areas 11.

### Reference Signs List

1 ... Garment, 10 ... Insulation area, 100 ... Contact surface, 11 ... Electrode area, 110 ... Contact surface , 111 ... Conductive composite thread, 1110 ... Elastic fiber, 1111 ... Conductive fiber, 1111a ... Lower winding thread, 1111b ... Upper winding thread, 12 ... Wire, 13 ... Terminal, 14 ... Insulation member, 15 ... Fixing member, 2 ... Transceiver, 21 ... Control unit, 22 ... Storage unit, 23 ... Communication unit, 26 ... Terminal, 27 ... Fixing member, 3 ... Terminal, 31 ... Control unit, 32 ... Storage unit, 33 ... Communication unit, 34 ... Display unit, 35 ... Operation unit, 4 ... Disposable garment, 40 ... Insulation area, 400 ... Contact surface, 401 ... Recess, 41 ... Electrode area, 410 ... Contact surface, 42 ... Wire, 43 ... Terminal, 46 ... Hook-and-loop fastener, 47 ... Pocket, 470 ... Inlet port, 48 ... Opening, 5 ... Communication network, 8 ... Garment, 80 ... Insulation area, 800 ... Contact surface, 81 ... Electrode area, 810 ... Contact surface, 82 ... Wire, 83 ... Terminal, 84 ... Insulation member, 9 ... Detection system, 9a ... Detection system, D1 ... Diameter, L ... Line, O ... Center line, P ... Wearer

## Claims

1. A garment comprising:
an insulation area formed by weaving a piece of insulating fabric that insulates electricity; and
an electrode area formed by weaving a thread that contains a piece of conductive fiber made by adding a conductive material to a piece of non-conductive fiber, such that a difference in height is not formed between the electrode area and the insulation area,
wherein the electrode area is provided at a position that comes into contact with the body of a wearer such that a biopotential is detected when a wearer wears the garment.

2. The garment according to claim 1,
wherein the thread that contains the piece of conductive fiber is a conductive composite thread formed by winding the piece of conductive fiber around a piece of elastic fiber that has a stretch ratio of at least 50%.

3. The garment according to claim 2,
wherein the piece of insulating fabric is an insulating composite thread formed by winding the piece of non-conductive fiber around the piece of elastic fiber.

4. The garment according to claim 3,
wherein a winding number of the piece of non-conductive fiber in the insulating composite thread is the same as a winding number of the piece of conductive fiber in the conductive composite thread.

5. The garment according to any one of claims 1 to 4,
wherein the electrode area is provided at two positions that are separated from each other by the insulation area.

6. The garment according to claim 5, further comprising:
a receiver that receives the biopotential detected by each of the electrode areas provided at the two positions;
wires that respectively extend from the electrode areas;
two terminals that are respectively provided at end portions of the wires, and are connected to the receiver; and
a fixing member that is provided away from a line that connects the two terminals to each other, and fixes the receiver to the insulation area.

7. The garment according to any one of claims 1 to 6,
wherein the electrode area is provided at a position at which a skin between a chest and abdomen of the wearer contacts with the electrode area.

8. A conductive composite thread formed by winding a piece of conductive fiber around a piece of elastic fiber that has a stretch ratio of at least 50%, the piece of conductive fiber being formed by adding silver to a piece of non-conductive fiber.

9. The conductive composite thread according to claim 8,
wherein the non-conductive fiber is nylon and the elastic fiber is polyurethane.

10. A disposable garment comprising:
a piece of non-woven cloth that has a pocket and an opening;
an electrode that is formed from the conductive composite thread according to claim 8 or 9, and is bonded to the piece of non-woven cloth;
a receiver housed in the pocket; and
a wire that connects the electrode to the receiver,
wherein the disposable garment is to be worn by a wearer such that a side, to which the electrode is bonded, of the piece of non-woven cloth faces the front surface of the body of the wearer, and the head of the wearer passes through the opening.

11. The disposable garment according to claim 10,
wherein the pocket is located on the left side of the front surface when the disposable garment is worn by the wearer.

12. The disposable garment according to claim 10 or 11,
wherein the pocket has an inlet port on the lower edge side when the disposable garment is worn by the wearer, and
the receiver is to be inserted into the pocket from the inlet port.
